# EUROPEAN PATENT SPECIFICATION

(11) **EP 0 316 764 B1**
(45) Date of publication and mention of the grant of the patent: **08.06.1994**
(21) Application number: 88118727.2
(22) Date of filing: 10.11.1988
(51) Int. Cl.: G01N 33/574, C12Q 1/34, G01N 33/543, G01N 33/577, C12P 21/00, C12N 5/00, C12N 15/00

(54) **Detection of tumor-associated markers in biological fluids**
Nachweis von Tumorassozierten Anzeigern in biologischen Flüssigkeiten
Détection de marqueurs associés aux tumeurs dans des fluides biologiques

(30) Priority: 20.11.1987 US 123439
(43) Date of publication of application: 24.05.1989
(73) Proprietor: ABBOTT LABORATORIES, Abbott Park, Illinois 60064-3500 (US)
(72) Inventor: Kinders, Robert, Arlington Heights, IL 60004 (US); Konrath, John, Waukegan, IL 60087 (US); Slota, John, Grayslake, IL 60030 (US); Rittenhouse, Harry George, Lake Bluff, IL 60044 (US); Voight, Julie, Gurnee, IL 60031 (US)
(74) Representative: Modiano, Guido, Dr.-Ing.

(56) References cited:
- EP-A- 0 225 709
- EP-A- 0 313 244
- WO-A-87/01392
- CHEMICAL ABSTRACTS, vol. 75, no. 21, 22 November 1971, Columbus, OH (US); J.L. VAITUKAITIS et al., p. 17, no. 126325b#
- JOURNAL OF THE NEUROLOGICAL SCIENCES, vol. 70, 1985, Elsevier Science Publishers B.V., Amsterdam (NL); P. GALLO et al., pp. 81-92#
- JOURNAL OF BIOLOGICAL CHEMISTRY, vol. 256, no. 21, 10 November 1981, Baltimore, MD (US); W.W. YOUNG, Jr. et al., pp. 10967-10972#
- CHEMICAL ABSTRACTS, vol. 102, no. 21, 27 May 1985, Columbus, OH (US); E.F. HOUNSELL et al., p. 448, no. 183507n#

## Description

The invention relates to an improved immunoassay method for detection of cryptic tumor-associated (CTA) antigens in biological fluids. Particularly, the invention is an immunoassay method for detection of CTA antigens in body fluids which incorporates sample digestion with neuraminidase to unmask tumor-associated epitopes prior to testing for the CTA antigens.

Diagnostic immunoassays for tumor-associated antigens are well known in the art. For example, immunoassays are commercially available for detection of various tumor-associated antigens such as carcinoembryonic antigen (CEA), alpha-fetoprotein (AFP), CAl25, CAl9-9 and CAl5-3. These immunoassays detect the presence of tumor-associated antigens in biological fluids such as serum, plasma or urine.

One problem associated with the presently available assays is that they do not detect tumor-associated antigens at very early stages of disease. Another problem is that many of the immunoassays are not sensitive or specific enough to detect a particular antigen in all samples. Therefore, many of the available assays are used only for monitoring levels of particular antigens in known cancer patients as a patient management tool or for research use. It is therefore highly desirable to develop new assay procedures which will detect tumor-associated antigens at an earlier stage of disease and to increase specificity and sensitivity of these immunoassays.

In U.S. Patent No. 4,116,776 to Dalbow et al. it is taught that malignant neoplastic cells synthesize and secrete human chorionic gonadotropin (hCG) into the circulatory system where the glycoprotein hormone is collected or adsorbed by the host's lymphocytes. Dalbow et al. state that the prior art tested the wrong fraction of blood, i.e., serum instead of lymphocytes and was not sufficiently sensitive. Dalbow et al. disclose an improved method for detection of hCG in which white blood cells are isolated from blood samples and pretreated with neuraminidase followed by testing for the presence of hCG.

Similarly, in PCT Patent Application No. WO 87/01392 to Coulter Corporation, it is disclosed that detection of human carcinoma tumor antigens in a pathological sample can be enhanced by removing sialic acid from the antigen's determinant site using neuraminidase. As in the Dalbow et al. patent, Coulter teaches treatment of a cell or tissue preparation with neuraminidase prior to assay for presence of tumor antigens.

Also of relevance to the present invention is EP-A-0 313 244 which falls within the terms of Article 54(3) EPC. EP-A- 0 313 244 discloses a method for enhancing immunological binding to a target ligand (e.g., cryptic tumor-associated mucin antigens) in a body fluid sample comprising treating the ligand with neuraminidase prior to performing an immunoassay.

### SUMMARY OF THE INVENTION

The present invention is an improved method of detection of cryptic tumor-associated (CTA) antigens in body fluids such as serum, plasma, pleural effusion, sputum, urine, nipple discharge or feces. The method incorporates neuraminidase treatment of the sample without any necessity for prior preparation of cells. The neuraminidase treatment removes sialic acid to unmask CTA epitopes and frees the CTA antigens for reaction with antibody reagents resulting in a more sensitive and specific immunoassay.

### DETAILED DESCRIPTION OF THE INVENTION

### Brief Description of the Figures.

Figure 1 shows results of testing of documented normal human serum and blood donors using the CTA antigen assay of Example 1;
Figure 2 shows results of testing of blood donors, benign breast disease, early breast cancer and Stage IV breast cancer sera using the CTA antigen assay of Example 1;
Figure 3 illustrates a comparison of the CTA assay of Example 1 against a commercially available assay;
Figure 4 shows a comparison of the CTA assay of Example 2 against a commercially available assay for a documented normal population;
Figure 5 shows a comparison of the CTA assay of Example 2 against a commercially available assay for another normal population; and
Figure 6 shows a comparison of the CTA assay of Example 2 against a commercially available assay for Stage IV breast cancer sera.

In the improved method for detection of CTA antigens, body fluid samples such as serum, plasma, whole blood, urine or pleural effusions are treated with neuraminidase in order to expose CTA antigens. As used herein, "cryptic tumor-associated antigens" or "CTA antigens" shall mean any tumor-associated antigen which is hindered or prevented from reacting fully with antibodies specific for the antigens due to interference with sialic acid which may be attached to the CTA antigens or located in close spatial proximity to specific CTA epitopes so as to interfere with binding of the antigens by certain antibodies. Once the sialic acid is removed by neuraminidase treatment, the CTA antigens are free to react with antibodies in an immunoassay procedure. In the method according to the invention, the treatment of the body fluid test sample with neuraminidase is performed simultaneously with the first step of the immunoassay. The assay can be a homologous or a heterologous format, that is, the same or different antibodies may be used on the solid phase and labelled phase to detect the tumor-associated antigen. Further, the assay can be configured as direct or competitive formats and the assay can be a one-step assay or may preferably be accomplished in two or more steps.

In one assay format, an antibody (polyclonal or monoclonal) specific for a tumor-associated antigen is adsorbed or covalently bound onto a solid phase such as polystyrene beads, microparticles, wells, strips, plates or other suitable plastic or paper solid supports which are well known to those skilled in the art. The solid phase antibody is referred to herein as the anchor antibody. In a first incubation, the anchor antibody is used to bind a specific tumor-associated antigen from a body fluid sample. The sample is treated with neuraminidase simultaneously with the first incubation, i.e., the sample is added to the anchor antibody and treated with neuraminidase in a first step.

Next, any antigen in the sample which is not bound by the anchor antibody and any residual serum components and neuraminidase are removed by washing. Then the tumor-associated antigen in the sample is reacted in a second incubation with a labelled probe antibody which may be the same or different from the anchor antibody. Labels which are useful in the assay include enzymes, radioisotopes and fluorescent labels and any other chemical or biological entity which will produce a detectable signal and are well known to those skilled in the art. Preferably, the labelled probe is an antibody-enzyme conjugate such as antibody-horseradish peroxidase.

Unbound labelled probe antibody is removed by washing, and then the label is detected in the solid phase as a measure of the presence or amount of the tumor-associated antigen. If an enzyme is used as the label, a substrate is added which forms a colored product in the presence of the enzyme label. For example, if horseradish peroxidase is used, o-phenylenediamine is added as a substrate to form a colored product which can be measured visually or by spectrophotometric determination at an absorbance of 492 nm.

In a second assay format, a labelled antibody specific for a tumor-associated antigen is mixed with a body fluid sample suspected of containing a tumor-associated antigen. The sample is treated simultaneously with neuraminidase and the labelled antibody in the first incubation. After incubation, a solid phase such as microparticles coated with a tumor-associated antigen is added to the neuraminidase-treated mixture in order to digest the antigen on the solid phase so that any labelled antibody not bound by the sample can be bound to the solid phase antigen. The neuraminidase treatment of the solid phase antigen is critical to the assay because digestion of the solid phase antigen increases immunoreactivity of the antigen with the labelled antibody. The solid phase is then separated from the other components of the assay as by centrifugation, washing, filtration or a combination of these in order to remove any sample antigen-labelled antibody complexes, leaving only solid phase-bound labelled antibody . Next, a substrate for the label is added to the solid phase to produce a detectable signal such as fluorescence or color. The more sample antigen-antibody complex formed, the lower the amount of labelled antibody bound to the solid phase and the lower the color or fluorescence formed in the reaction.

The neuraminidase used to treat the sample may be from any source well known in the art. A preferred source of neuraminidase is obtained from Clostridium perfringens such as C. perfringens Type X from Sigma Chemical, St. Louis, Missouri.

The following examples illustrate the present invention.

### EXAMPLE 1

This example describes a cryptic tumor-associated antigen assay utilizing neuraminidase treatment of the sample.

### A. Preparation of Monoclonal Antibodies to Cryptic Tumor-Associated Antigen

A mucin glycoprotein immunogen was isolated and purified by S400 polyacrylamide gel permeation chromatography and high performance liquid chromatography (HPLC) from the ascites fluid of a pool of several cancer patients manifesting a variety of cancer types. The purified mucin glycoprotein was used to inject BALB/c mice three times using a RIBI adjuvant, RIBI Immunochem Research, Inc., Hamilton, Montana, with a final intravenous boost with neuraminidase-treated glycoprotein. The mice were sacrificed and spleens removed for fusion with myeloma cells such as SP2/0. The hybridoma cells were cultured in hypoxanthine aminoptern thymidene (HAT) medium, and hybrids were screened against the purified glycoprotein and neuraminidase-treated glycoprotein, red blood cell ghosts, white blood cells and a meconium glycosphingolipid preparation. The antibody so obtained, designated Abbott Laboratories 85/34, was determined to be a mouse IgM monoclonal antibody. This antibody was reactive with Paragloboside (PG) (Galβ1, 4-GlcNAcβ1, 3-Galβ1, 4-Glcβ1,1 Ceramide) and with mucinous glycoproteins prepared from ascitic fluids. Reaction with desialylated sialosyl α(2,3) lacto-N-norhexaosylceramide and sialosyl α(2,3) lacto-N-isooctaosylceramide suggested specificity for the Galβ1, 4-GlcNAcβ1, R sequence similar to the I or i antigen determinants.

The above antibody has been described in J. Cell. Biol., Abstracts of 16th Annual Meetings, U.C.L.A. Symposia on Molecular & Cellular Biology, Supp. 11D, p.157 (March 29 - May 1, 1987). Another similar antibody has been described by Young, et al., J. Biol. Chem., 256 (21):10967-10972 (1981). It is believed that any antibody having similar specificity to the antibodies described above which are specific for the N-acetyllactosamine family of oligosaccharides will work as well. The 85/34 antibody was used as an anchor antibody for the cryptic tumor-associated antigen assay.

### B. Preparation of F36/22 Antibody/Enzyme Conjugate.

A monoclonal antibody designated F36/22 was conjugated to horseradish peroxidase by the periodate method of Nakane and Pierce, J. Histochem. Cytochem., 22:1084-1091 (1974). The F36/22 antibody was obtained from Health Research, Inc., Roswell Park Division, Buffalo, New York, and is described in detail in Croghan et al., Cancer Research, 43:4980-4988 (1983); Cancer Research, 43:1741-1747 (1983); and Canadian Patent No. 1,215,331 issued on December 16, 1986. The antibody produced by the F36/22 hybridoma cell line is available through the American Type Culture Collection, Rockville, Maryland under ATCC Number HB 8215.

### C. Cryptic Tumor-Associated Antigen Assay Procedure.

0.1 ml of the 85/34 antibody (10-30 »g/ml in 10mM Tris, pH 7.4) was coated by passive adsorption at 37°C for one hour onto wells of plastic microtiter plates (Immulon II available from Dynatech, Inc., Alexandria, Virginia). Unbound antibody is removed by aspiration and washing with distilled water. The coated wells were then overcoated by addition of 2% bovine serum albumin (BSA) in phosphate buffered saline (PBS). Next, 50 »l of a diluent buffer containing 0.05% BSA in PBS plus thimerisol, gentamycin, and 0.5 mg/ml taurodeoxycholate (TDC-sodium salt, Sigma Chemical Company, St. Louis, Missouri) were added to each well.

10 »l of patient's serum is then added to each well, followed by addition of 50 »l of the diluent containing 100 mU/ml neuraminidase (C. perfringens, Type X, Sigma Chemical Co.). One unit of neuraminidase is defined as that amount liberating 1.0 micromole N-acetyl neuraminic acid per minute at pH 5.0 at 37°C using the manufacturer-indicated substrate. The coated wells are incubated with the serum, buffer and neuraminidase for 1 hour at 37°C. Unbound serum components are removed from the wells by aspiration and washing with distilled water.

0.1 ml of an approximately 100 ng/ml solution of F36/22 antibody/HRPO conjugate (diluted with 20% fetal calf serum, 0.1 M Tris buffer, 0.15M NaCl, and Gentamycin) is added to the wells and incubated at 37°C for one hour. Unbound conjugate is removed by washing ten times with distilled water. Next, an o-phenylenediamine.2HCl (OPD) substrate is added and incubated for twenty minutes in the dark, which forms a yellow product in the presence of the enzyme, followed by addition of 1 N H₂S0₄ to stop the reaction. The sample reaction is read on a spectrophotometer at 492 O.D.

### EXAMPLE 2

This example is included for illustrative purposes only. The method herein described provides teachings useful for understanding the putting into practice of the present invention, although it should be noted that part D of this example falls outside the present invention in that the patient's serum sample is pretreated with neuraminidase, whereas according to the present invention the pretreatment with neuraminidase should occur simultaneously with the addition of the 85/34 antibody-alkaline phosphatase conjugate in part D.

This example illustrates a competitive assay approach for cryptic tumor-associated antigens utilizing treatment of the sample with neuraminidase to free the cryptic tumor-associated antigens for reaction.

### A. Preparation of 85/34 Antibody-Alkaline Phosphatase Conjugate.

An 85/34 antibody-alkaline phosphatase conjugate was made as follows:
2 ml of 50 mM NaI0₄ is prepared by adding 21.4 mg NaIO₄ plus 2 ml of 0.01 M Na₂Co₃, pH 9.5. Equal volumes of periodate solution and alkaline phosphatase (10 mg/ml), i.e., 300 »l alkaline phosphatase and 300 »l periodate, are added together and incubated at room temperature for 2 hours. A 9 ml G-25 Column, Pharmacia Biotechnology Products, Piscataway, New Jersey, is equilibrated with 0.01 M Na₂CO₃, pH 9.5. The activated alkaline phosphatase is then passed over the column and approximately 1 ml fractions are collected and read at 280 nm for collection of the first fraction at this reading.

The concentration of the activated alkaline phosphatase enzyme is adjusted to 1 mg/ml using 0.01 M Na₂CO₄, assuming an extinction coefficient of one. Next, 1 M MgCl₂ and 0.1 M ZnCl₂ are added to a final concentration of 1 mM and 0.1 mM, respectively.

The activated alkaline phosphatase enzyme is conjugated to the 85/34 antibody described in Example 1 using a molar ratio of 1:1. The antibody and enzyme are incubated together for 4 to 6 hours at room temperature in the dark. Next, 10 mg NaBH₄ and 2 ml 0.01M NaCO₃ (pH 9.5) are added. 35 »l of NaBH₄ solution is then added for each ml of reaction solution. This is incubated overnight at 4°C. Next, a volume of acetone equal to 1/10 of the NaBH₄ volume is added. Finally, the conjugate is diluted with a diluent comprising 0.1 M Tris, 0.5 M NaCl, 20% calf serum and 3% Triton® to bring the antibody concentration to 100 »g/ml. Another diluent which also works well is 12 mg/ml BSA in PBS, pH 7.4. The antibody-alkaline phosphatase conjugate is stored at -20°C.

### B. Preparation of Microparticles.

A 2.5% solution of latex microparticles (0.21 »m commercially available from Seradyn, Indianapolis, Indiana) was made by diluting 41.66 ml microparticles in 458.34 ml distilled water. The microparticles were prewashed in an ion exchange media using a monosized mixed bed resin by mixing the diluted microparticles and the resin at a 2:1 ratio (500 ml particles:250 g resin). The particle/resin mixture is rotated at room temperature for 1 hour and then filtered through a coarse scintered glass funnel. The resin is then washed with distilled water 2-3 times but with no more than 400 ml water. The particles are then centrifuged at 10,000 rpm for 20 minutes, the supernatant poured off and the particles resuspended in distilled water to a final volume of 500 ml.

1 ml of the prewashed 2.5% microparticles is mixed at room temperature with 1 ml of 0.2 mg/ml carbodiimide (EDAC) (0.1 g EDAC, Sigma Chemical Co., St. Louis, Missouri, in 50 ml MES buffer described below) and 2 ml 5 mM MES buffer. The MES buffer is made by mixing 0.8 g of 5 mM MES, Sigma Chemical, with 800 ml distilled water and adjusting the pH to 4.75 with 6N NaOH.

### C. Antigen-Coating of Microparticles.

The microparticle mixture is vortexed for 10 seconds, and then a 1:5 dilution of a DUl45 antigen preparation (S300 gel permeation chromatography-purified fraction obtained from culture media) in PBS is added. The DU145 cell line is available from the American Type Culture Collection, Rockville, Maryland under ATCC# HTB-81E and is a human prostate carcinoma, metastasis to brain, described in detail in Internatl. J. Cancer, 21:274-281 (1978). This cell line is grown in 90% Eagle's minimum essential medium with 10% fetal bovine serum.

The microparticles plus DU145 antigen are vortexed for 5 seconds at low speed and then rotated for 2 hours at room temperature followed by centrifugation at 10,000 rpm for 30 minutes. Next, the supernatant is carefully removed, taking care not to disturb the antigen-coated microparticle pellet, and the pellet is washed with a PBS/Tween® solution by adding 1 ml PBS/Tween® and breaking up the pellet into a uniform suspension using a vortex and a pipette tip. This wash step is repeated five times and then the washed microparticles are pulled through a 25 g needle. The microparticles are then resuspended to 10 ml in PBS/Tween® and centrifuged at 10,000 rpm for 20 minutes.

The pellet is again washed in PBS/Tween® as described above and centrifuged at 10,000 rpm for 15 minutes. Next, the supernatant is removed with care not to disturb the pellet. Then the pellet is washed by adding 1 ml of an overcoat buffer consisting of 50 mM Tris, 100 mM NaCl, 0.1% BSA, pH 8.0 and breaking up the pellet in a uniform suspension using a vortex and a pipette tip. The overcoat buffer is added in increments of 1 ml up to 5 ml while continuing to mix the microparticles with the pipette and vortexing. The mixture is brought up to 10 ml with PBS/Tween® and stored overnight at 45°C. The microparticle mixture is centrifuged again at 10,000 rpm for 30 minutes, followed by another series of washing steps as outlined above. Finally, the washed microparticles are resuspended by adding 1 ml portions of PBS/Tween® buffer while breaking up the pellet into a uniform suspension with a pipette and vortex and bringing the final volume to 10 ml. The coated microparticles are stored at 2-8°C.

### D. Inhibition Assay for Cryptic Tumor-Associated Antigens.

50 »l of a diluent buffer containing Tris, 12.1 g/l; EDTA, 0.336 g/l; NaCl, 29.22 g/l; BSA, 0.5 g/l and distilled water containing 250 mU/ml neuraminidase (C. perfringens, Type X, Sigma Chemical Co.) is added to 50 »l of a patient's serum sample (diluted 1:10 in diluent buffer) and incubated for 5 minutes. Next, 100 »l of the 85/34 antibody-alkaline phosphatase conjugate is added and incubated for 25 minutes, followed by addition of 80 »l of DU145 coated microparticles. The reaction solution is incubated for 20 minutes at 34°C and then a methylylumbelliferone fluorophore substrate (MUP) which fluoresces in the presence of alkaline phosphatase is added. Fluorescence is measured at 448 nm.

One instrument particularly useful in performing the above-described assay and measuring the amount of fluorescence is the IMX™ instrument available from Abbott Laboratories, Abbott Park, Illinois. The amount of fluorescence is inversely proportional to the amount of cryptic tumor antigen in the sample, i.e., a low amount of fluorescence is indicative of a high amount of antigen in the sample and vice versa.

### EXAMPLE 3

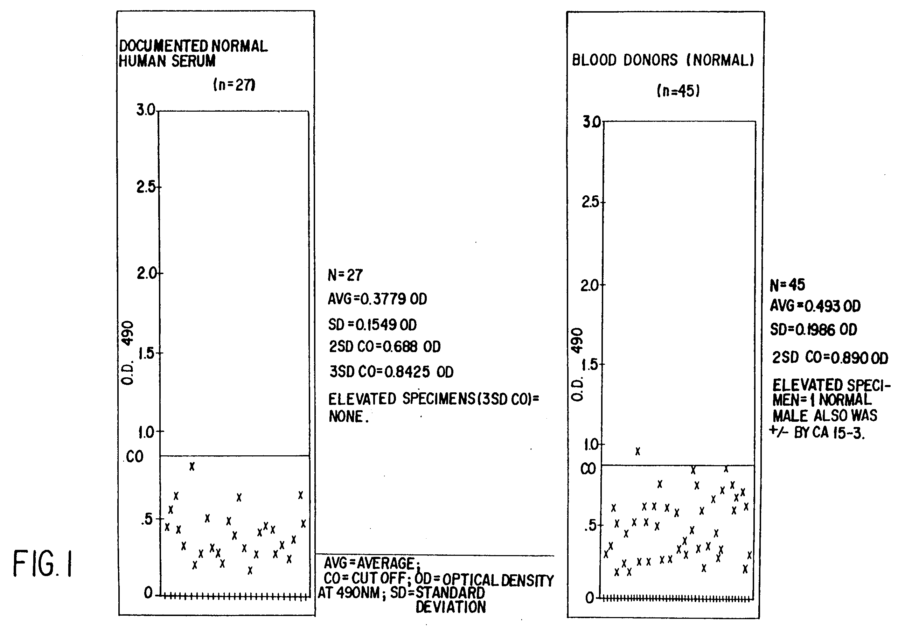

The following Figures 1-6, illustrate the results of testing using the CTA assays described in Examples 1 and 2. Specifically, Figures 1, 2 and 3 show results of testing using the Example 1 assay. The cutoff for all assays (Figures 1, 2 and 3) was established as the mean of the optical density (O.D.) at 490 nm plus two standard deviations from the mean, of the Blood Donors (Normal) population, i.e., 0.890 O.D. That cutoff was selected for optimal specificity of the assay. Figure 1 demonstrates that none of a group of documented normal human sera was positive by the CTA assay of Example 1, and that only 1 out of 45 normal blood donor sera tested positive. This positive sample was also borderline positive when tested by another commercially available research use assay (Centacor CA 15-3, Centacor, Malvern, Pennsylvania).

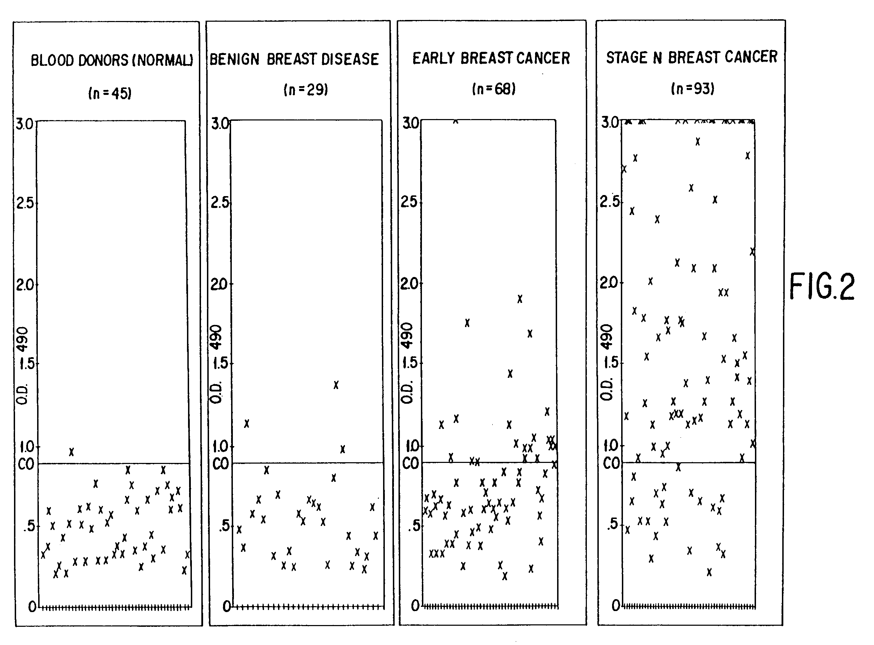

Figure 2 demonstrates testing of benign breast disease, early breast cancer and Stage IV breast cancer in human sera compared to the normal blood donor population shown in Figure 1. As can be seen in this figure, 3 of 29 benign breast disease sera, 21 of 68 early breast cancer sera and 72 of 93 Stage IV breast cancer sera tested positive by the CTA assay.

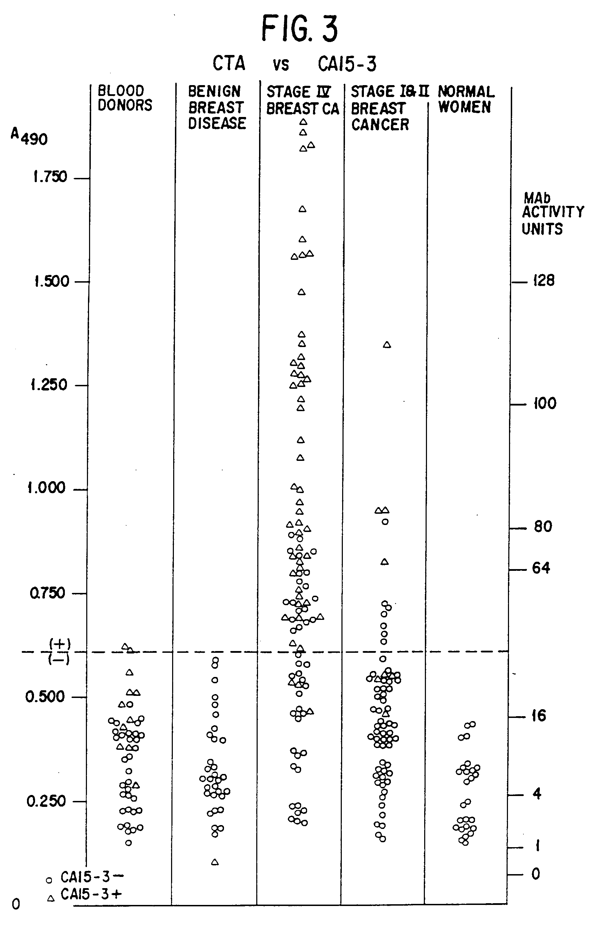

Figure 3 shows a head-to-head comparison of the CTA assay to the CA 15-3 assay. The 67 sera are from a documented specimen panel obtained from the University of Toronto Medical Center, Toronto, Canada and the Wisconsin Clinical Cancer Center, Madison, Wisconsin. Normal women were documented by breast examination, family histories and normal menstrual cycles. The horizontal dashed line represents the cut-off (with a background of 0.2 subtracted) separating persons with normal levels of the CTA marker from elevated levels associated with cancer, and is based on the mean plus two standard deviations of the blood donor population. The cutoff level chosen is greater than the mean plus three standard deviations of the documented FIG. 3 Normal Women population to enhance specificity. CA 15-3 negative specimens are indicated by circles, CA 15-3 positive specimens are indicated by triangles. As shown, none of the 26 documented normal samples were positive by either the CTA assay or the CA 15-3 assay. However, the CA 15-3 assay showed 11 of 67 blood donor samples to be positive, while the CTA assay showed only 1 of 67 of these samples to be positive. The CA 15-3 assay resulted in a positive result for 1 of 32 benign breast disease samples, while the CTA assay found none of these samples to be positive. Finally, the CA 15-3 assay found 8 of 66 Stage I and II breast cancer samples to be positive and 49 of 92 Stage IV breast cancer samples positive, while the CTA assay determined 11 of 66 of the Stage I and II samples to be positive and 66 of 92 of the Stage IV samples to be positive.

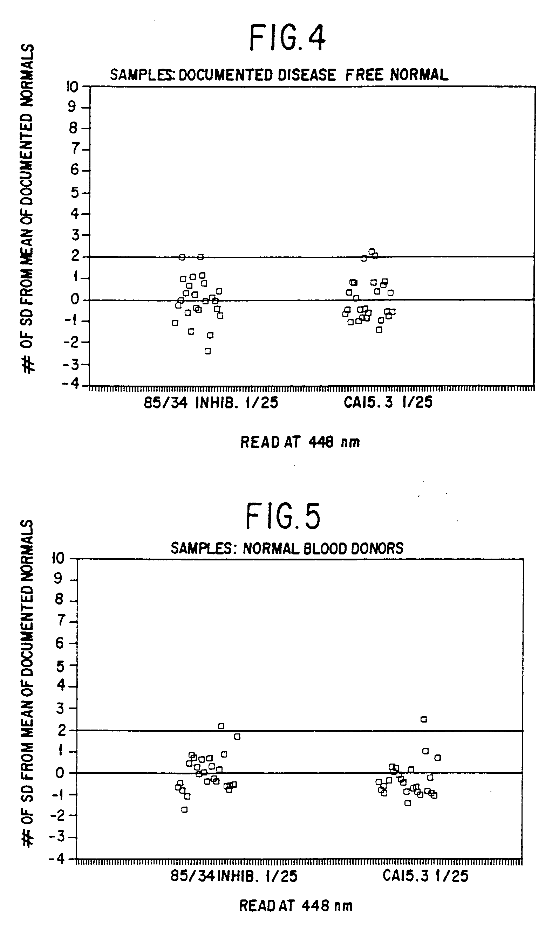

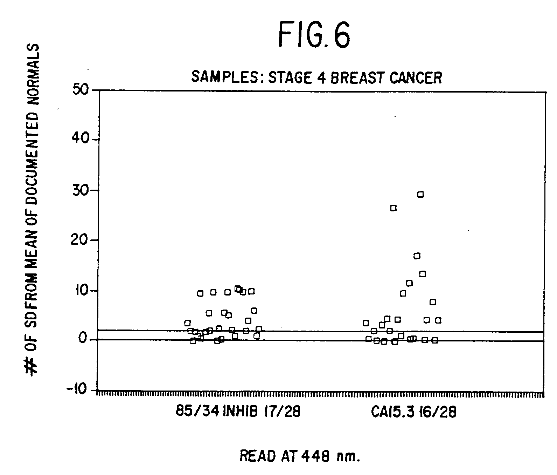

Figures 4-6 show test results using the CTA assay of Example 2. Figure 4 shows a comparison of the CTA assay of Example 2 with the CA 15-3 assay. In testing 26 documented normal human sera (disease free) the CTA assay found only one sample to be positive, while the CA 15-3 assay found two positives. Testing on an additional normal population (Figure 5) showed that the CTA assay and the CA 15-3 assay each detected 1 of 25 samples as positive. For stage IV breast cancer samples (Figure 6), the CTA assay detected 17 of 28 samples as positive, while the CA 15-3 assay detected 16 of 28.

The neuraminidase treatment of a sample in the assay for cryptic tumor antigens offers many advantages. First, antigens in a patient sample which previously could not be detected or could only be detected in insignificant amounts can now be detected due to unmasking of the antigen for reaction. Second, the antigens are freed up by the neuraminidase treatment for a more specific reaction with the antibody of choice in the assay. Third, the cryptic tumor antigen assay of the invention is more sensitive than previous assays as evidenced by greater detection of tumor-associated antigens in cancer patients than prior art assays. Fourth, the assay has a lower false positive rate than prior art assays. Finally, the assay is convenient and simple to perform.

As a final note, it may be mentioned that it is theorized that removal of sugar moieties from other tumor-associated antigens with enzymes appropriate for digestion of those sugar linkages may enhance detection of many other tumor-associated antigens.

## Claims

1. A method for detection of cryptic tumor-associated antigens in body fluid samples comprising:
a. treating a body fluid sample with neuraminidase to free up the cryptic tumor-associated antigens for reaction with an antibody specific for the cryptic tumor-associated antigens; and
b. testing the neuraminidase-treated sample by immunoassay for the cryptic tumor-associated antigens,
wherein steps a. and b. are performed simultaneously.

2. A method according to claim 1 for detection of a cryptic tumor-associated antigen in a body fluid sample comprising:
a. adsorbing an antibody specific for the cryptic tumor-associated antigen onto a solid phase;
b. treating the sample with neuraminidase;
c. adding the sample to the antibody-coated solid phase and incubating;
d. washing the solid phase to remove any unreacted sample;
e. adding a labelled antibody specific for the cryptic tumor-associated antigen and incubating;
f. washing the solid phase to remove any unreacted labelled antibody; and
g. detecting the label as a measure of the presence or amount of tumor-associated antigen in the sample,
wherein steps b. and c. are performed simultaneously.

3. The method of claim 2 wherein the solid phase antibody is a monoclonal antibody reactive with Paragloboside (PG) (Galβ1, 4-GlcNAcβ1, 3-Galβ1, 4-Glcβ1, 1 Ceramide) and the N-acetyllactosamine family of oligosaccharides.

4. The method of claim 2 wherein the labelled antibody is an F36/22 monoclonal antibody produced by an F36/22 hybridoma cell line of the American Type Culture Collection, Rockville, Maryland under ATCC Number HB 8215.

5. A method according to claim 1 for detecting a cryptic tumor-associated antigen in a body fluid sample comprising:
a. treating the sample with neuraminidase;
b. adding a labelled antibody to the sample, the antibody being specific for a tumor-associated antigen, and incubating;
c. adding a solid phase coated with a tumor-associated antigen to the sample and incubating;
d. separating the solid phase from the liquid phase; and
e. detecting the label on the solid phase as a measure of the presence or amount of tumor-associated antigen in the sample,
wherein steps a. and b. are performed simultaneously.

6. The method of claim 5 wherein the labelled antibody is a monoclonal antibody reactive with with Paragloboside (PG) Galβ1, 4-GlcNAcβ1, 3-Galβ1, 4-Glcβ1, 1 Ceramide) and the N-acetyllactosamine family of oligosaccharides.

7. The method of claim 5 wherein the solid pahse antigen is a DU145 antigen preparation obtained from a DU145 cell line available from the American Type Culture Collection, Rockville, Maryland under ATCC# HTB-81E.

## Patentansprüche

1. Verfahren zum Nachweis kryptischer, mit einem Tumor in Verbindung stehender Antigene in Körperflüssigkeitsproben, umfassend:
a. Behandeln einer Körperflüssigkeitsprobe mit Neuraminidase, um die kryptischen, mit einem Tumor in Verbindung stehenden Antigene zur Reaktion mit einem Antikörper freizusetzen, der für die kryptischen, mit einem Tumor in Verbindung stehenden Antigene spezifisch ist; und
b. Testen der mit Neuraminidase behandelten Probe durch einen Immuntest auf die kryptischen, mit einem Tumor in Verbindung stehenden Antigene,
bei dem die Stufen a und b gleichzeitig durchgeführt werden.

2. Verfahren nach Anspruch 1 zum Nachweis eines kryptischen, mit einem Tumor in Verbindung stehenden Antigens in einer Körperflüssigkeitsprobe, umfassend:
a. Adsorbieren eines Antikörpers, der für das kryptische, mit einem Tumor in Verbindung stehende Antigen spezifisch ist, auf einer festen Phase;
b. Behandeln der Probe mit Neuraminidase;
c. Hinzugeben der Probe zu der antikörperbeschichteten festen Phase und Inkubieren;
d. Waschen der festen Phase, um nicht umgesetzte Probe zu entfernen;
e. Zugeben eines markierten Antikörpers, der für das kryptische, mit einem Tumor in Verbindung stehende Antigen spezifisch ist und Inkubieren;
f. Waschen der festen Phase, um nicht umgesetzten markierten Antikörper zu entfernen; und
g. Nachweisen der Markierung als Maß für das Vorliegen oder die Menge des mit einem Tumor in Verbindung stehenden Antigens in der Probe, bei dem die Stufen b und c gleichzeitig durchgeführt werden.

3. Verfahren nach Anspruch 2, bei dem der Festphasen-Antikörper ein monoklonaler Antikörper ist, der mit Paraglobosid (PG) (Galβ1, 4-GlcNAcβ1, 3-Galβ1, 4-Glcβ1, 1-Ceramid) und der N-Acetyllactosaminfamilie der Oligosaccharide reaktiv ist.

4. Verfahren nach Anspruch 2, bei dem der markierte Antikörper ein monoklonaler F36/22-Antikörper ist, der von einer F36/22-Hybridomzellinie aus der American Type Culture Collection, Rockville, Maryland, ATCC-Nummer HB 8215, produziert wird.

5. Verfahren nach Anspruch 1 zum Nachweis eines kryptischen, mit einem Tumor in Verbindung stehenden Antigens in einer Körperflüssigkeitsprobe, umfassend:
a. Behandeln der Probe mit Neuraminidase;
b. Zugeben eines markierten Antikörpers zu der Probe, wobei der Antikörper für ein mit einem Tumor in Verbindung stehendes Antigen spezifisch ist, und Inkubieren;
c. Zugeben einer festen Phase, die mit einem mit einem Tumor in Verbindung stehenden Antigen beschichtet ist, zu der Probe und Inkubieren;
d. Trennen der festen Phase von der flüssigen Phase und
e. Nachweisen der Markierung auf der festen Phase als Maß für das Vorliegen oder die Menge eines mit einem Tumor in Verbindung stehenden Antigens in der Probe,
bei dem die Stufen a. und. b. gleichzeitig durchgeführt werden.

6. Verfahren nach Anspruch 5, bei dem der markierte Antikörper ein monoklonaler Antikörper ist, der mit Paraglobosid (PG) (Galβ1, 4-GlcNAcβ1, 3-Galβ1, 4-Glcβ1, 1-Ceramid) und der N-Acetyllactosaminfamilie der Oligosaccharide reaktiv ist.

7. Verfahren nach Anspruch 5, bei dem das Festphasenantigen eine DU145-Antigenpräparation darstellt, die aus einer DU145-Zellinie erhalten worden ist, die von American Type Culture Collection, Rockville, Maryland unter der ATCC-Nummer HTB-81E erhältlich ist.

## Revendications

1. Méthode pour la détection des antigènes associés aux tumeurs cryptiques dans des échantillons de fluide corporel comprenant :
a. le traitement d'un échantillon de fluide corporel par une neuraminidase pour libérer les antigènes associés aux tumeurs cryptiques de façon à leur permettre de réagir avec un anticorps spécifique des antigènes associés aux tumeurs cryptiques ; et
b. le test de l'échantillon traité par la neuraminidase par un dosage immunologique pour les antigènes associés aux tumeurs cryptiques,
dans laquelle les étapes a. et b. sont réalisées de façon simultanée.

2. Méthode selon la revendication 1 pour la détection d'un antigène associé à une tumeur cryptique dans un échantillon de fluide corporel comprenant :
a. l'adsorption d'un anticorps spécifique de l'antigène associé à une tumeur cryptique sur une phase solide ;
b. le traitement de l'échantillon par la neuraminidase ;
c. l'addition de l'échantillon à la phase solide revêtue de l'anticorps et la mise en incubation ;
d. le lavage de la phase solide, pour éliminer l'échantillon qui n'aurait pas réagi ;
e. l'addition d'un anticorps marqué spécifique de l'antigène associé à une tumeur cryptique et la mise sous incubation ;
f. le lavage de la phase solide pour éliminer l'anticorps qui n'aurait pas réagi ; et
g. la détection du marqueur comme mesure de la présence ou de la quantité de l'antigène associé à une tumeur dans l'échantillon,
dans laquelle les étapes b. et c. sont réalisées de façon simultanée.

3. Méthode selon la revendication 2, dans laquelle l'anticorps en phase solide est un anticorps monoclonal réactif pour le Paragloboside (PG) (Galβ1, 4-GlcNacβ1, 3-Galβ1, 4-Glcβ1, 1 Céramide) et la famille des N-acétyllactosamines des oligosaccharides.

4. Méthode selon la revendication 2, dans laquelle l'anticorps marqué est un anticorps monoclonal F36/22 produit par une lignée cellulaire d'hybridomes F36/22 de l'American Type Culture Collection, Rockville, Maryland sous le numéro ATCC HB 8215.

5. Méthode selon la revendication 1 pour la détection d'un antigène associé à une tumeur cryptique dans un échantillon de fluide corporel comprenant :
a. le traitement de l'échantillon par la neuraminidase ;
b. l'addition d'un anticorps marqué à l'échantillon, l'anticorps étant spécifique d'un antigène associé à une tumeur et la mise en incubation ;
c. l'addition à l'échantillon d'une phase solide revêtue d'un antigène associé à une tumeur et la mise en incubation ;
d. la séparation de la phase solide de la phase liquide ; et
e. la détection du marqueur sur la phase solide comme mesure de la présence ou de la quantité de l'antigène associé à une tumeur dans l'échantillon,
dans laquelle les étapes a. et b. sont réalisées de façon simultanée.

6. Méthode selon la revendication 5, dans laquelle l'anticorps marqué est un anticorps monoclonal réactif pour le Paragloboside (PG) (Galβ1, 4-GlcNacβ1, 3-Galβ1, 4-Glcβ1, 1 Céramide) et la famille des N-acétyllactosamines des oligosaccharides.

7. Méthode selon la revendication 5, dans laquelle l'antigène en phase solide est une préparation d'antigène DU145 obtenue à partir d'une lignée de cellules DU145 disponible dans l'American Type Culture Collection, Rockville, Maryland sous le numéro ATCC HTB-81E.
